# EUROPEAN PATENT APPLICATION

(11) **EP 1 518 856 A1**
(43) Date of publication of application: **30.03.2005**
(21) Application number: 03728079.9
(22) Date of filing: 16.05.2003
(51) Int. Cl.: C07D 401/04, C07D 209/54

(54) **PROCESS FOR PRODUCING QUINOLONECARBOXYLIC ACID DERIVATIVE**

(30) Priority: 17.05.2002 JP 2002142383
(71) Applicant: DAIICHI PHARMACEUTICAL CO., LTD., Chuo-ku, Tokyo 103-8234 (JP)
(72) Inventor: OHTA, N.,C/O DAIICHI PHARM. CO., LTD. TOKYO R&D C., Tokyo 134-8630 (JP); AKIBA, T.,C/O DAIICHI PHARM. CO., LTD. TOKYO R&D C, Tokyo 134-8630 (JP)
(74) Representative: Hartz, Nikolai F., Dr.
(86) International application number: PCT/JP2003/006119
(87) International publication number: WO 2003/097634

(57) **Abstract**

Through a production process according to the following reaction scheme, compounds (2) which are useful for antibacterial agents can be provided at low cost and high yield.

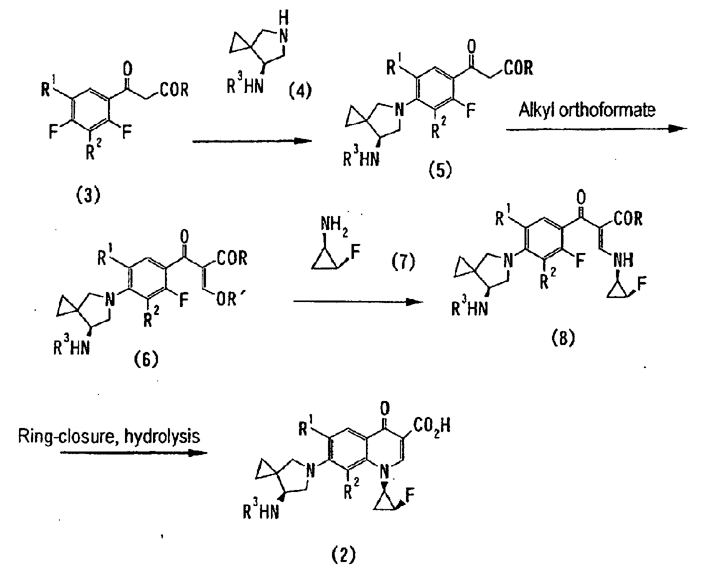

## Description

### Technical Field

The present invention relates to a process for producing an intermediate for producing quinolonecarboxylic acid derivatives having high safety and antibacterial activity, and to an intermediate which is a key compound in the process.

### Background Art

Quinolonecarboxylic acid derivatives are widely used as synthetic antibacterial drugs in the medical field. However, resistant bacteria and side effects in relation to the derivatives are critical issues in the medical treatment. In order to solve the problems, a variety of quinolonecarboxylic acid derivatives have been synthesized, and antibacterial activity and safety of the derivatives have been studied.

The following compounds represented by formula (I) exhibit strong antibacterial activity (Japanese Patent No. 2714597). 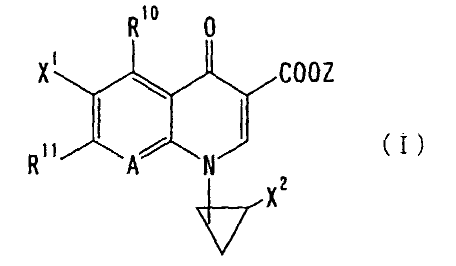 [wherein R¹⁰ represents an amino group, a methylamino group, a hydroxyl group, a thiol group, or a hydrogen atom; R¹¹ represents a substituent represented by the following formula: 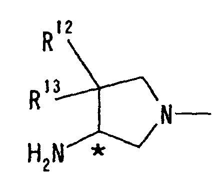 (wherein R¹² and R¹³ form a methylene chain by linking with each other to form a 3- to 6-membered ring) or a 3-hydroxypyrrolidinyl group whose 4-position carbon atom is spiro-linked with cyclopropane; A represents C-X³ (wherein X³ represents a halogen atom, a C1-C6 alkyl group, a C1-C6 alkoxy group, a trifluoromethyl group, or a hydrogen atom) or a nitrogen atom; each of X¹ and X² independentlyrepresents a halogen atom; and Z represents a hydrogen atom, a C1-C6 alkyl group, a C1-C6 alkoxyalkyl group, a phenylalkyl group having a C1-C6 alkyl chain, a phenyl group, an acetoxymethyl group, a pivaloyloxymethyl group, an ethoxycarbonyloxy group, a choline group, a dimethylaminoethyl group, a 5-indanyl group, a phthalidinyl group, a 5-substituted-2-oxo-1,3-dioxol-4-ylmethyl group, or a 3-acetoxy-2-oxobutyl group].

Among the compounds represented by formula (I), particularly, the following compounds (1): 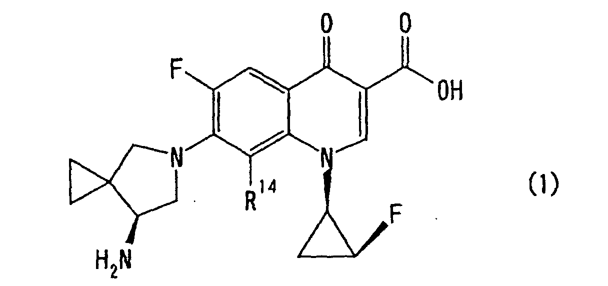 (wherein R¹⁴ represents a methoxy group or a chlorine atom) not only possesses strong antibacterial activity, but is also excellent in low toxicity and high safety, so this compound is a compound expected to be able to overcome problems associated with resistant bacteria and safety.

Taking a compound (1) in which R¹⁴ is a methoxy group as an example, an already known process for producing these compounds will next be described, with reference to the following reaction cascade: 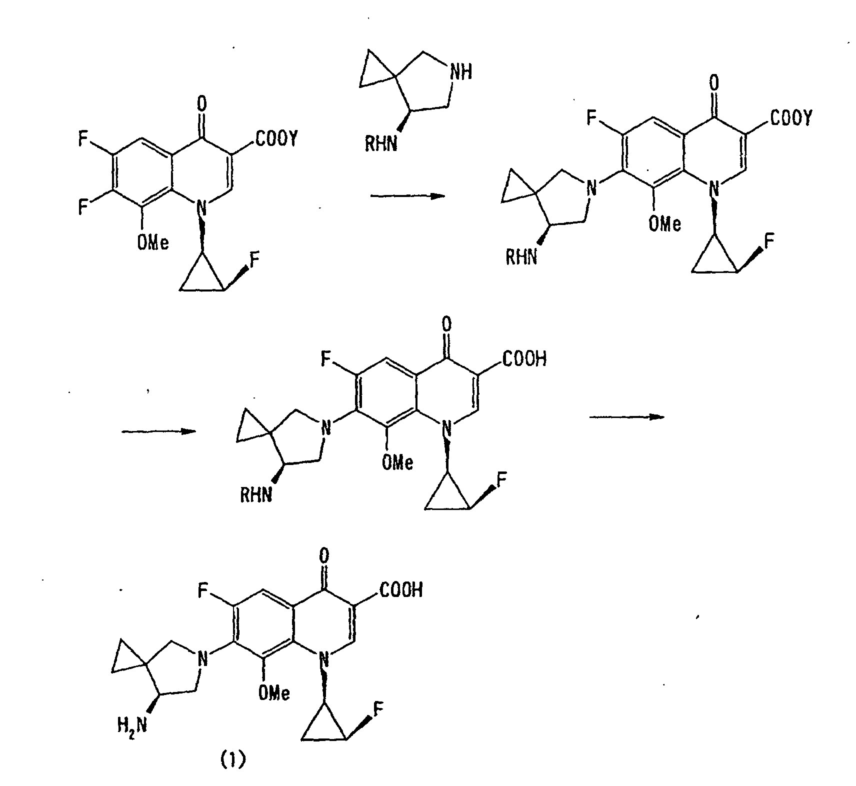 (wherein R represents an amino-group-protective group, though this definition has nothing to deal with the present invention).

In this reaction scheme, a pyrrolidinyl group is introduced into quinolone skeletone compound by the substitution at the 7^{th} position of the quinolone skeletone compound having a fluorocyclopropylamino moiety at position 1.

At the initial stage, the following compound (7) is to be incorporated as the substituent at position 1 of the quinolone skeletone.

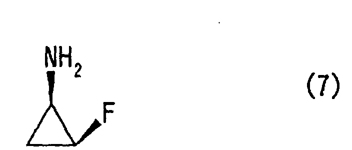

However, this reaction process has some problems such that the production cost of compound (7) is extremely expensive; this compound needs to be introduced at the initial stage; and the production cost of compound (1) grows more expensive as a result. Thus, a solution is urgently needed for the decrese of total production cost by introducing the reaction step of compound (7) at a later stage.

In view of the foregoing, an object of the present invention is to provide an industrially advantageous process for producing compounds (2a): 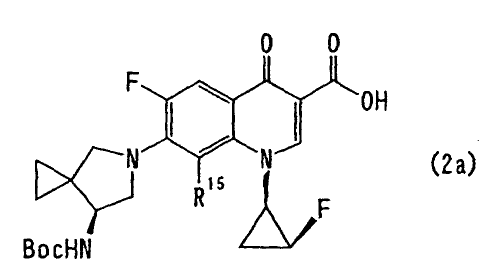 (wherein R¹⁵ represents a methoxy group or a hydrogen atom), which serve as intermediates for producing the aforementioned quinolonecarboxylic acid derivatives (1) having excellent antibacterial activity and high safety. Another object of the invention is to provide an intermediate playing a vital part in the production process.

### Disclosure of the Invention

The present inventors have carried out extensive researches seeking a production process that is capable of more efficiently producing intermediates (2a) needed to obtain the quinolonecarboxylic acid delivative (1). As a result, it has been found that such intermediates, including intermediate (2a), can be produced more advantageously in the industrial scale if a pyrrolidine compound is reacted with a compound represented by formula (3) to introduce a pyrrolidine substituent into the compound; then the foregoing compound (7) is reacted with said pyrrolidine-substituted compound just before a quinolone ring is formed by the ring-closure reaction. Thus, the present invention was accomplished.

Accordingly, the present invention provides:
a process for producing a compound represented by formula (2): 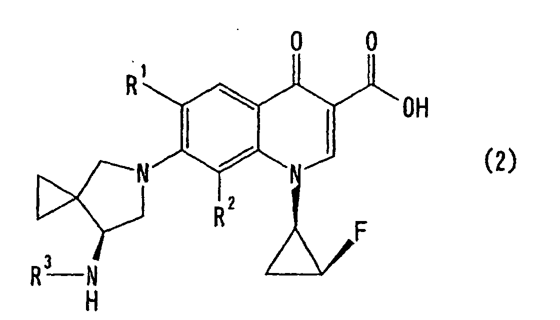 (wherein each of R¹ and R² independently represents a hydrogen atom, a fluorine atom, a chlorine atom, a C1-C6 alkyl group, or a C1-C6 alkoxy group; and R³ represents a hydrogen atom or an amino-group-protective group), the process comprising:
reacting a compound represented by formula (3): 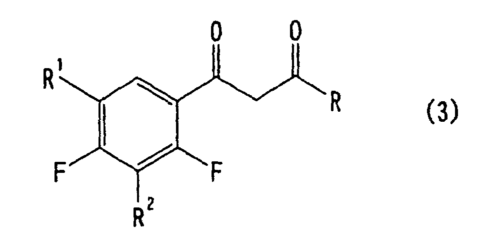 (wherein R represents an aryloxy group, an aralkyloxy group, or a C1-C6 alkoxy group; and R¹ and R² have the same meanings as defined above) with a compound represented by formula (4): 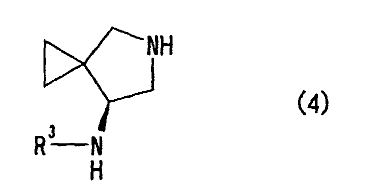 (wherein R³ has the same meaning as defined above), to thereby yield a compound represented by formula (5): 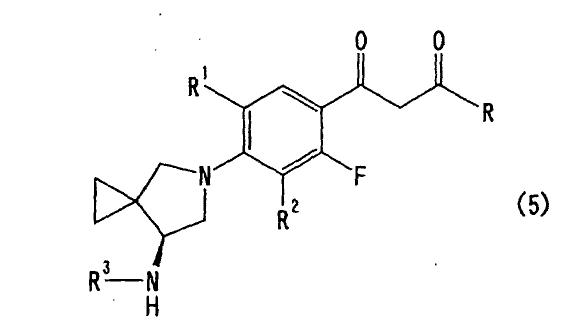 (wherein R, R¹, R², and R³ have the same meanings as defined above);
reacting the compound represented by formula (5) with an alkyl orthoformate, to thereby yield a compound represented by formula (6): 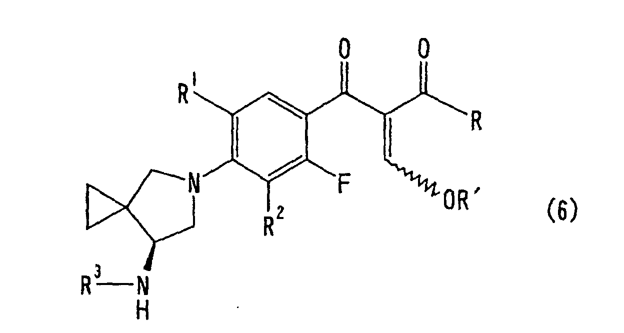 (wherein R' represents a C1-C6 alkyl group; and R, R¹, R², and R³ have the same meanings as defined above);
reacting the compound represented by formula (6) with a compound represented by formula (7): 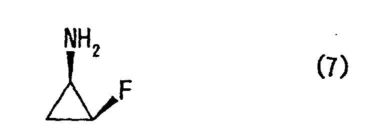 to thereby yield a compound represented by formula (8): 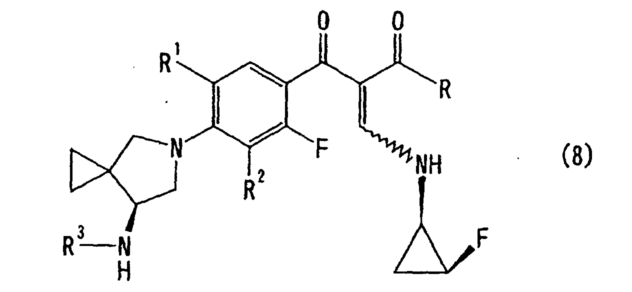 (wherein R, R¹, R², and R³ have the same meanings as defined above);
performing ring closure of the compound represented by formula (8); and
performing ester hydrolysis of the formed ring-closed compound.

The invention also provides:
a process for producing a compound represented by formula (2): 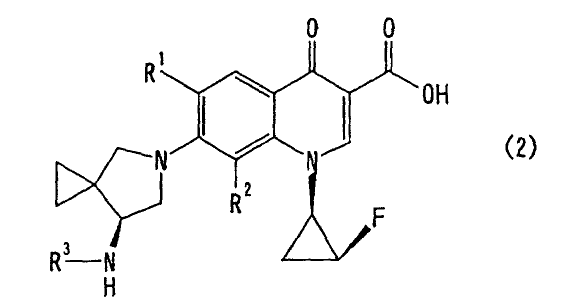 (wherein R¹, R², and R³ have the same meanings as defined above) comprising performing ring closure of a compound represented by formula (8): 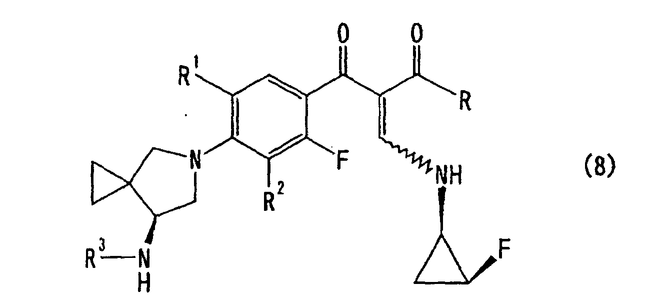 (wherein R, R¹, R², and R³ have the same meanings as defined above) and performing ester hydrolysis of the formed ring-closed compound.

The present invention also provides a compound represented by formula (5): 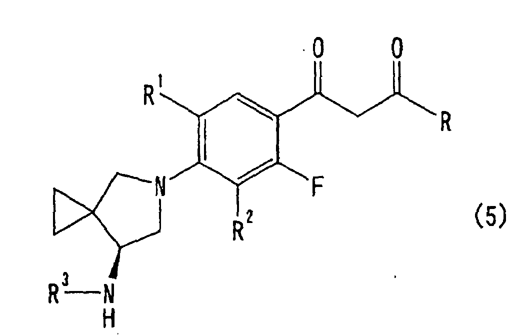 (wherein R represents an aryloxy group, an aralkyloxy group, or a C1-C6 alkoxy group; each of R¹ and R² independently represents a hydrogen atom, a fluorine atom, a chlorine atom, a C1-C6 alkyl group, or a C1-C6 alkoxy group; and R³ represents a hydrogen atom or an amino-group-protective group); a salt thereof; a hydrate of the compound (5); or a hydrate of the salt.

The present invention also provides a compound represented by formula (6): 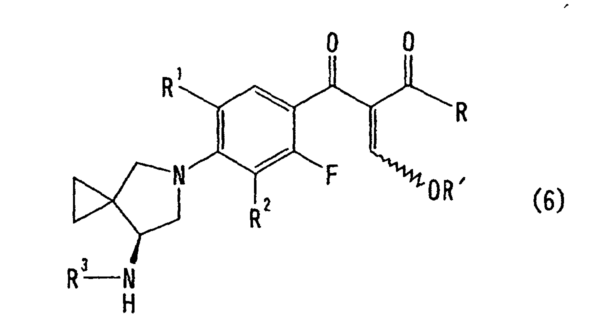 (wherein R represents an aryloxy group, an aralkyloxy group, or a C1-C6 alkoxy group; each of R¹ and R² independently represents a hydrogen atom, a fluorine atom, a chlorine atom, a C1-C6 alkyl group, or a C1-C6 alkoxy group; R³ represents a hydrogen atom or an amino-group-protective group; and R' represents a C1-C6 alkyl group); a salt thereof; a hydrate of the compound (6); or a hydrate of the salt.

The invention also provides a compound represented by formula (8): 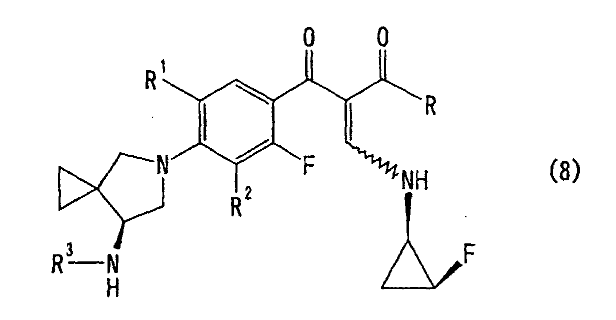 (wherein R represents an aryloxy group, an aralkyloxy group, or a C1-C6 alkoxy group; each of R¹ and R² independently represents a hydrogen atom, a fluorine atom, a chlorine atom, a C1-C6 alkyl group, or a C1-C6 alkoxy group; and R³ represents a hydrogen atom or an amino-group-protective group); a salt thereof; a hydrate of the compound (8); or a hydrate of the salt.

### Best Mode for Carrying Out the Invention

The compound (2) can be produced from the compound (3) through the following steps: 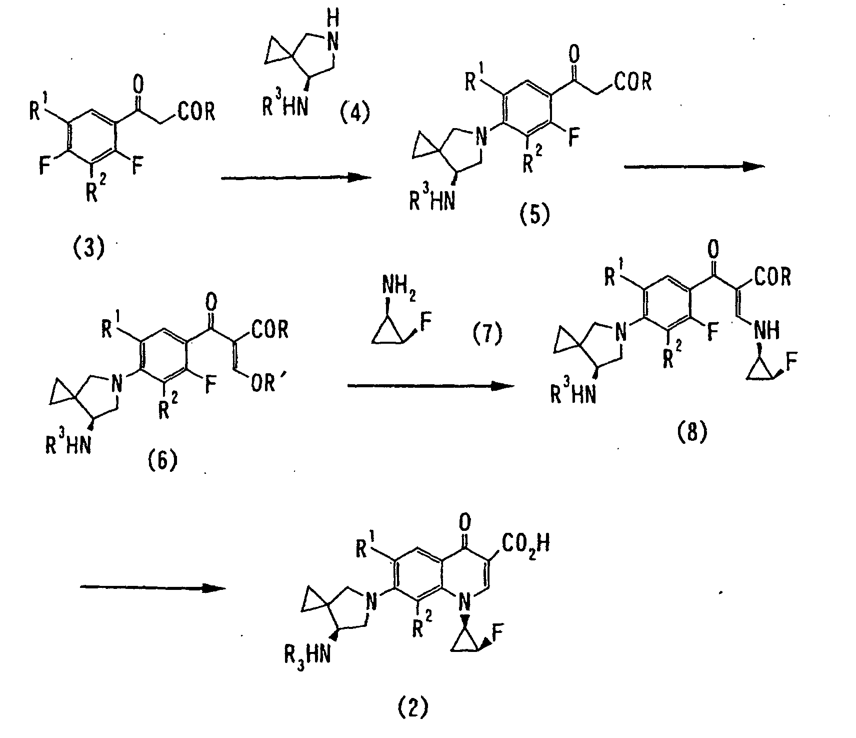 (wherein R¹, R², R³, R, and R' have the same meanings as defined above).

In the compound (3), each of R¹ and R² independently represents a hydrogen atom, a fluorine atom, a chlorine atom, a C1-C6 alkyl group, or a C1-C6 alkoxy group. No particular limitation is imposed on the selection of these substituents so long as the substituents do not inhibit reaction between the compound (3) and the amine compound (4). Among these substituents, a fluorine atom is preferred as R¹, and a methoxy group or a hydrogen atom are preferred as R².

In the amine compound (4), R³ represnts a hydrogen atom or an amino-group-protective group. The amino-group-protective group may be selected from the group consisting of an alkoxycarbonyl group, an aralkyloxycarbonyl group, an acyl group, an aralkyl group, an alkoxyalkyl group, and a substituted silyl group. No particular limitation is imposed on the type of amino-group-protective groups, and any groups can be employed so long as the groups do not inhibit reaction between the compound (3) and the amine compound (4) and can effectively be deprotected in the course of production of the compound (1). Of these, an alkoxycarbonyl group, an aralkyloxycarbonyl group, an acyl group, an alkoxyalkyl group, and a substituted silyl group are preferred as R³, with an alkoxycarbonyl group, an aralkyloxycarbonyl group, and an acyl group being more preferred.

Examples of the alkoxycarbonyl group include C1-C6 alkoxycarbonyl groups which may be substituted by one to three halogen atoms. Specific examples include a tert-butoxycarbonyl group (Boc group) and a 2,2,2-trichloroethoxycarbonyl group, with a tert-butoxycarbonyl group being preferred.

Examples of the aralkyloxycarbonyl group include C7-C20 aralkyloxycarbonyl groups which may be substituted by a C1-C6 alkoxy group or a nitro group. Specific examples include a benzyloxycarbonyl group (Cbz group), a p-methoxybenzyloxycarbonyl group, and a p-nitrobenzyloxycarbonyl group, with a benzyloxycarbonyl group (Cbz group) being preferred.

Examples of the acyl group include C2-C6 alkanoyl groups, a formyl group, and a benzoyl group which may be substituted by a C1-C6 alkoxy group, a halogen atom, etc. Specific examples include an acetyl group, a methoxyacetyl group, a trifluoroacetyl group, a chloroacetyl group, a pivaloyl group, a formyl group, and a benzoyl group, with an acetyl group being preferred.

Examples of the aralkyl group include C7-C20 aralkyl groups which may be substituted by a C1-C6 alkoxy group, a nitro group, etc. Specific examples include a 1-phenylethyl group, a benzyl group, a p-nitrobenzyl group, a p-methoxybenzyl group, and a triphenylmethyl group.

Examples of the alkoxyalkyl group include C1-C6 alkoxy-C1-C6 alkyl groups and C3-C5 cycloether groups which may be substituted by a halogen atom. Specific examples include a methoxymethyl group, a tert-butoxymethyl group, a 2,2,2-trichloroethoxymethyl group, and a tetrahydrofuranyl group.

Examples of the substituted silyl group include tri-C1-C6 alkylsilyl groups, tri-C7-C20 aralkylsilyl groups, and C1-C6 alkyldiphenylsilyl groups. Specific examples include a trimethylsilyl group, an isopropyldimethylsilyl group, a tert-butyldimethylsilyl group, a tribenzylsilyl group, and a tert-butyldiphenylsilyl group.

No particular limitation is imposed on the type of R, and any groups can be employed so long as the groups can form a carboxylic ester. Examples of such Rs include aryloxy groups, aralkyloxy groups, and C1-C6 alkoxy groups. Specific examples include a phenyloxy group, a benzyloxy group, methoxy group, an ethoxy group, and a propoxy group. These groups may further contain a substituent such as a halogen atom or an alkyl group. Among them, a C1-C6 alkyl group is preferably employed from the viewpoint of simplicity. Of these, a methyl group and an ethyl group are preferred.

The step for yielding the compound (5) will next be described. The step is accomplished by reacting the compound (3) with the amine compound (4). The amine compound (4) employed may be a free base or a salt with an inorganic acid or an organic acid. Preferably, the amine compound is used in an amount of one equivalent or more.

The step is preferably performed in the presence of a base. This is because HF is formed in this step along with the progress of reaction, and the formed HF may inhibit reaction with the compound (3) by forming a salt with the amine compound from which an essential protective group is removed; may corrode a reactor tank; or may raise a problem of environmental pollution. In order to prevent such problems, the step is preferably performed in the presence of a base. The base is preferably used in an amount of one equivalent or more. When an acid-added salt of the compound (4) is used in the step, an additional base is required in order to convert the salt to the corresponding free base.

Examples of salts of the amine compound (4) include salts of an inorganic acid such as hydrochloric acid, sulfuric acid, nitric acid, hydrofluoric acid, hydrobromic acid, or hydriodic acid; and salts of an organic acid such as p-toluenesulfonic acid, methanesulfonic acid, trifluoroacetic acid, acetic acid, formic acid, maleic acid, or fumaric acid. These salts may assume hydrate form or solvate form.

Examples of the base employed in the step include organic bases such as trimethylamine, triethylamine, and 4-(dimethylamino)pyridine and inorganic bases such as ammonia, potassium carbonate, sodium carbonate, sodium hydroxide, and potassium hydroxide. Of these, tertiary amines, *inter alia,* triethylamine, are preferred.

No particular limitation is imposed on the reaction solvent so long as the solvent does not inhibit reaction. Examples include acetonitrile, N,N-dimethylacetamide, N,N-dimethylformamide, dimethyl sulfoxide, and N-methylpyrrolidone, with acetonitrile and N,N-dimethylacetamide being preferred.

The reaction temperature can be arbitrarily selected from a range of the solidification temperature to the boiling temperature of the reaction mixture, preferably selected from a range of room temperature to the boiling temperature of the reaction mixture. The reaction time, which is a period before complete consumption of starting material being confirmed, is generally one hour to 100 hours, preferably one hour to 24 hours.

The step for yielding the compound (6) will next be described. The step is performed by reacting the compound (5) with an alkyl orthoformate in acetic anhydride. Although the compound (5) is not always required to be an isolated and purified form, such a form is preferred. Preferably, alkyl orthoformate and acetic anhydride are each used in an amount of one equivalent or more.

The alkyl orthoformate used in the step may have a C1-C6 alkyl group. Of these, ethyl orthoformate and methyl orthoformate are preferred. Notably, the alkyl moiety of the alkyl orthoformate forms R' of the compound (6). In other words, R' is preferably an ethyl group or a methyl group.

In order to promote reaction, an inorganic acid such as sulfuric acid; an organic acid such as acetic acid; or a Lewis acid such as zinc chloride may be added as a catalyst.

No particular limitation is imposed on the reaction solvent so long as the solvent does not inhibit the reaction. Preferably, an alkyl orthoformate is used as a reagent and solvent.

The reaction temperature can be arbitrarily selected from a range of the solidification temperature to the boiling temperature of the reaction mixture, preferably selected from a range of room temperature to the boiling temperature of the solvent. The reaction time, though this time corresponds to a period before complete consumption of starting material being confirmed, is generally one hour to 100 hours, preferably one hour to six hours.

The step for yielding the compound (8) will next be described. The step is performed by reacting the compound (6) with the amino compound (7) in a solvent in the presence of a base. Although the compound (6) is not always required to be an isolated and purified form, such a form is preferred.

Examples of the base include organic bases such as trimethylamine, triethylamine, and 4-(dimethylamino)pyridine; and inorganic bases such as ammonia, potassium carbonate, sodium carbonate, sodium hydroxide, and potassium hydroxide. Of these, tertiary amine is preferred, and triethylamine is particularly preferred. When the amine compound (7) is an acid-added salt, the base is preferably used in an amount required for converting the salt to the corresponding free base plus at lease in an amount required for capturing hydrogen fluoride generated during reaction.

The compound (7) may be an acid-added salt. The acid which forms such an acid-added salt may be an inorganic acid or an organic acid. Examples include inorganic acids such as hydrochloric acid, sulfuric acid, nitric acid, hydrofluoric acid, hydrobromic acid, and hydriodic acid; and organic acids such as p-toluenesulfonic acid, benzenesulfonic acid, and methanesulfonic acid (i.e., sulfonic acids which may optionally have a substituent such as a halogen atom or an alkyl group) and trifluoroacetic acid, acetic acid, formic acid, maleic acid, and fumaric acid (i.e., carboxylic acids).

No particular limitation is imposed on the reaction solvent, and any solvents can be used so long as the solvents does not inhibit reaction. Examples include toluene, N,N-dimethylacetamide, N,N-dimethylformamide, dimethyl sulfoxide, and N-methylpyrrolidone. Of these, toluene is preferred.

The reaction temperature can be arbitrarily selected from a range of the solidification temperature to the boiling temperature of the reaction mixture. However, room temperature is preferred. The reaction time, though this time corresponds to a period before complete consumption of starting material being confirmed, is generally 30 minutes to 24 hours, preferably 30 minutes to six hours.

The step for yielding the compound (2) will next be described. The step is performed by reacting the compound (8) in a solvent in the presence of a base. In addition, a phase-transfer catalyst may be used in combination. Although the compound (8) is not always required to be an isolated and purified form, such a form is preferred.

Examples of the base include organic bases such as trimethylamine, triethylamine, and 4-(dimethylamino)pyridine; and inorganic bases such as ammonia, potassium carbonate, sodium carbonate, sodium hydroxide, and potassium hydroxide. Of these, potassium hydroxide is preferred. The base is preferably used at least in an amount required for capturing hydrogen fluoride generated during ring closure and for hydrolyzing the formed ester. The base may be directly added to reaction system or may be added in the form of solution obtained by dissolving the base in a solvent such as water. Preferably, an aqueous solution of the base is added. The base or the solution thereof does not necessarily form an admixture with the reaction solvent.

No particular limitation is imposed on the reaction solvent so long as the solvent does not inhibit reaction. Examples include toluene, N,N-dimethylacetamide, N,N-dimethylformamide, dimethyl sulfoxide, and N-methylpyrrolidone. Of these, toluene is preferred.

No particular limitation is imposed on the phase-transfer catalyst so long as the catalyst promotes reaction. Among such catalysts, tetrabutylammonium bromide (TBAB) is preferred.

The reaction temperature can be arbitrarily selected from a range of the solidification temperature to the boiling temperature of the reaction mixture, preferably selected from a range of room temperature to the boiling temperature of the reaction mixture. The reaction time, though this time corresponds to a period before complete consumption of starting material being confirmed, is generally one hour to 100 hours, preferably one hour to 24 hours.

In the process of the present invention, the compound (2) can be isolated and purified by an ordinary method. Specifically, in one method, the pH of the reaction mixture is adjusted through addition of an appropriate acid thereto, and the mixture was stirred under cooling with ice. The thus-precipitated crystals of the compound (2) are collected through filtration. In another method, the pH of the reaction mixture is adjusted through addition of an appropriate acid thereto, and an appropriate extraction solvent is added to the mixture, to thereby extract the compound (2). The obtained extract is concentrated, and the compound (2) is recrystallized from an appropriate extraction solvent.

In the aforementioned production process, the compound (2) may be yielded in a free form or a salt form. Examples of the salts include salts of an inorganic acid such as hydrochloric acid, sulfuric acid, nitric acid, hydrofluoric acid, hydrobromic acid, or hydriodic acid; salts of an organic acid such as p-toluenesulfonic acid, methanesulfonic acid, trifluoroacetic acid, trichloroacetic acid, acetic acid, formic acid, maleic acid, or fumaric acid; and salts of an alkali metal or an alkaline earth metal such as sodium, potassium, calcium, or lithium. Even when the compound (2) assumes a free form or a salt, the compound (2) may be yielded in the form of solvate thereof. The solvate may be formed by water, ethanol, propanol, acetonitrile, acetone, etc., or may be formed by absorbing water contained in air.

### Examples

The present invention will next be described in detail by way of Referential Examples and Examples.

### Referential Example 1: Ethyl 2,4,5-trifluoro-3-methoxybenzoylacetate

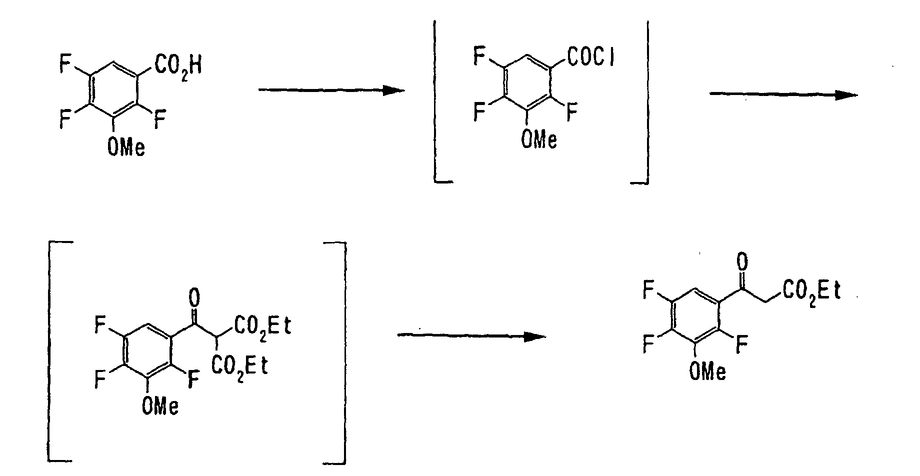

Thionyl chloride (5.6 mL, 1.5 eq.) was added to a solution containing 2,4,5-trifluoro-3-methoxybenzoic acid (10.00 g, 48.52 mmol), toluene (50 mL), and dimethylformamide (0.1 mL), and the mixture was stirred for one hour under reflux. After the reaction mixture had been allowed to cool, the solvent was removed under reduced pressure. Toluene (10 mL) was added to the residue, and the mixture was subjected to co-boiling for three times. After completion of co-boiling, toluene (50 mL) was added to the co-boiling residue, and diethyl malonate (7.77 g, 1.0 eq.) and magnesium ethoxide (11.10 g, 2.0 eq.) were added to the mixture, followed by stirring at 25°C for one hour. After the reaction mixture had been cooled, the organic layer was sequentially washed with sulfuric acid (19.0 g, 4.0 eq.)/water (50 mL) and saturated saline (50 mL). The washed organic layer was dried over sodium sulfate, and the solvent was removed under reduced pressure. Water (50 mL) and p-toluenesulfonic acid (120 mg) were added to the residue, and the mixture was stirred for 16 hours under reflux. After the reaction mixture had been allowed to cool, the mixture was subjected to extraction twice with toluene (30 mL), and the resultant organic layer was washed with sodium hydrogencarbonate (2.0 g)/water (30 mL) and with saturated saline (30 mL). The washed organic layer was dried over sodium sulfate, and the solvent was removed under reduced pressure, to thereby yield 11.87 g of the title compound as pale yellow oil (89%).
¹H-NMR (270 MHz, DMSO-d₆) δ: 1.18 (3H, t, J = 6.0 Hz), 4.02 (3H, s), 4.09 (2H, s), 4.12 (2H, q, J = 6.0 Hz, 8.0 Hz), 7.65(1H, m)
MASS: m/e = 276 (EIMS)

### Example 1: Ethyl 4-[7-(S)-tert-butoxycarbonylamino-5-azaspiro[2.4]hept-5-yl]-2,5-difluoro-3-methoxybenzoylacetate

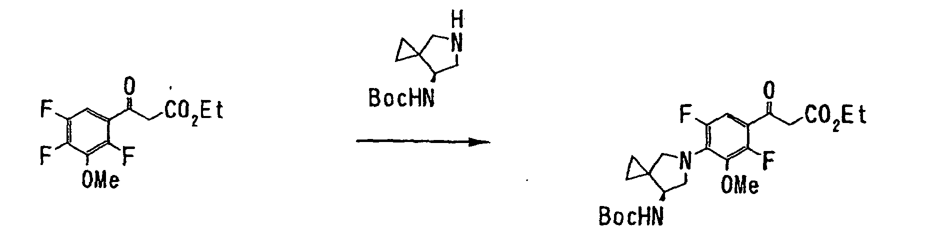

7-(S)-tert-Butoxycarbonylamino-5-azaspiro[2.4]heptane (3.84 g, 1.0 eq.) was added to a solution containing ethyl 2,4,5-trifluoro-3-methoxybenzoylacetate (5.00 g, 18.10 mmol), acetonitrile (50 mL), and triethylamine (5.1 mL, 2.0 eq.), and the mixture was sequentially stirred at 25°C for three days and at 50°C for four hours. After the reaction mixture had been allowed to cool, the solvent was removed under reduced pressure. Toluene (50 mL) was added to the residue, and the resultant organic solution (organic layer) was washed with water (30 mL) and saturated saline (30 mL). The organic layer was dried over sodium sulfate, and the solvent was removed under reduced pressure. The residue was subjected to silica gel column chromatography (eluent: hexane/ethyl acetate = 3/1), and several fractions were combined. The solvent of the combined fraction was removed under reduced pressure, to thereby yield 6.30 g of the title compound as yellow-green oil (74%).
¹H-NMR (270 MHz, CDCl₃) δ: 0.64-0.90 (4H, m), 1.29 (3H, t, J = 7.2 Hz), 1.35 (9H, s), 3.28-4.25 (5H, m), 3.81 (3H, s), 3.89 (2H, d, J = 3.5 Hz), 4.23 (2H, q, J = 7.2 Hz, 14.3 Hz), 7.34(1H, m)
MASS: m/e = 469 (EIMS)

### Example 2: Ethyl 3-ethoxy-2-[4-[7-(S)-tert-butoxycarbonylamino-5-azaspiro[2.4]hept-5-yl]-2,5-difluorobenzoyl-3-methoxy]acrylate

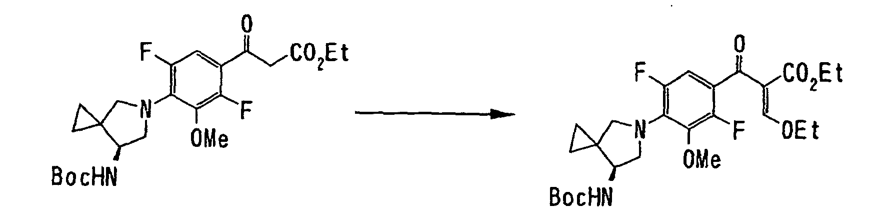

Ethyl 4-[7-(S)-tert-butoxycarbonylamino-5-azaspiro[2.4]hept-5-yl]-2,5-difluoro-3-methoxybenzoylacetate (1.00 g, 2.14 mmol) was dissolved in acetic anhydride (1.21 mL, 6 eq.) and ethyl orthoformate (2.13 mL, 6 eq.), and the mixture was stirred for 14 hours at 130°C. After the reaction mixture was allowed to cool down, the solvent was removed under reduced pressure. Toluene (10 mL) was added to the residue, and the mixture was subjected to co-boiling twice. After completion of co-boiling, the residue was subjected to silica gel column chromatography (eluent: hexane/ethyl acetate = 3.5/1), and several fractions were combined. Acetic acid contained in the liquid was neutralized with solid sodium bicarbonate, and the formed inorganic product was removed through filtration. The solvent of the filtrate was removed under reduced pressure, to thereby yield 790 mg of the title compound as a yellow-orange solid (71%).
¹H-NMR (270 MHz, CDCl₃) δ: 0.61-0.90 (4H, m), 1.19 (3H, t, J = 7.2 Hz), 1.21 (3H, t, J = 7.2 Hz), 1.39 (9H, s), 3.26-4.29 (5H, m), 3.78 (3H, s), 4.13 (2H, q, J = 7.2 Hz, 14.2 Hz), 4.17 (2H, q, J = 7.2 Hz, 14.2 Hz), 7.26 (1H, q, J = 6.9 Hz, 14.7 Hz), 7.59 (1H, s)
MASS: m/e = 525 (FABMS)

### Example 3: Ethyl 3-[(1R,2S)-2-fluoro-1-cyclopropylamino]-2-[4-[7-(S)-tert-butoxycarbonylamino-5-azaspiro[2.4]hept-5-yl]-2,5-difluorobenzoyl-3-methoxy]acrylate

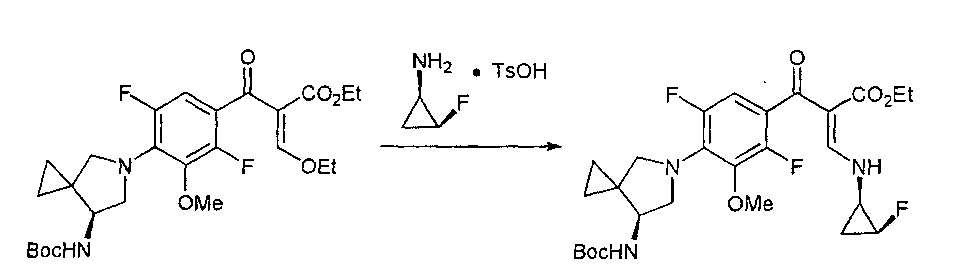

A (1R,2S)-2-fluorocyclopropylamine p-toluenesulfonic acid salt (359 mg, 1.0 eq.) was added to a solution containing ethyl 3-ethoxy-2-[4-[7-(S)-tert-butoxycarbonylamino-5-azaspiro[2.4]hept-5-yl]-2,5-difluorobenzoyl-3-methoxy]acrylate (760 mg, 1.45 mmol), toluene (15.2 mL), and triethylamine (0.22 mL, 1.1 eq.), and the mixture was stirred for 10 minutes at 25°C. The formed organic layer was washed with water (10 mL × 2) and saturated saline (10 mL) and dried over sodium sulfate. The solvent was removed under reduced pressure, to thereby yield 790 mg of the title compound as a yellow-orange (99%).
¹H-NMR (270 MHz, CDCl₃) δ: 0.61-1.31 (6H, m), 1.12 (3H, t, J = 7.0 Hz), 1.39 (9H, s), 3.18-4.13 (6H, m), 3.83 (3H, s), 4.10 (2H, q, J = 7.0 Hz, 10.3 Hz), 4.81 (1H, m), 7.25 (1H, m), 8.13 (1H, d, J = 13.8 Hz)
MASS: m/e = 554 (FABMS)

### Example 4: 7-[7-(S)-tert-Butoxycarbonylamino-5-azaspiro[2.4]hept-5-yl]-6-fluoro-1-[2-(S)-fluoro-1-(R)-cyclopropyl]-1,4-dihydro-8-methoxy-4-oxoquinoline-3-carboxylic acid

Tetrabutylammonium bromide (TBAB, 8 mg) was added to a solution containing ethyl 3-[(1R, 2S)-2-fluoro-1-cyclopropylamino]-2-[4-[7-(S)-tert-butoxycarbonylamino-5-azaspiro[2.4]hept-5-yl]-2,5-difluorobenzoyl-3-methoxy]acrylate (740 mg, 1.34 mmol), toluene (14.8 mL), and 3N potassium hydroxide (2.23 mL, 5 eq.), and the mixture was stirred at 50°C for four hours. 3N Potassium hydroxide (2.23 mL, 5 eq.) was further added to the mixture, and the resultant mixture was stirred at 50°C for two hours. After cooling of the mixture with ice, the mixture was slightly acidified with 3N aqueous hydrochloric acid, to thereby form a suspension. Water (15 mL) and saturated saline (5 mL) were added to the suspension so as to cause partition. The aqueous layer was subjected to extraction with toluene (20 mL × 2), to thereby recover organic components, and all the obtained organic layers were combined. The combined organic layer was dried over sodium sulfate, and the solvent was removed under reduced pressure. The residue was subjected to silica gel column chromatography (eluent: chloroform/methanol = 95/5), and several fractions were combined. The solvent of the combined fraction was removed under reduced pressure. The residue was dissolved in toluene (1.5 mL), and hexane (15 mL) was added to the solution. The mixture was stirred 25°C for three hours. The formed precipitates were collected through filtration and dried, to thereby yield 410 mg of the title compound as yellow-orange crystals (61%).
¹H-NMR (270 MHz, CDCl₃): Coincided with the ¹H-NMR spectrum of the title compound synthesized through a conventional method
MASS: Coincided with the data of the title compound synthesized through a conventional method (m/e = 506; FABMS)

### Example 5: Ethyl 4-[7-(S)-tert-butoxycarbonylamino-5-azaspiro[2.4]hept-5-yl]-2,5-difluorobenzoylacetate

7-(S)-[tert-Butoxycarbonylamino]-5-azaspiro[2.4]heptane (3.11 g, 1.2 eq.) was added to a solution containing ethyl 2,4,5-trifluorobenzoylacetate (3.00 g, 12.19 mmol), acetonitrile (60 mL), and triethylamine (3.4 mL, 2.0 eq.), and the mixture was stirred for 23 hours at 40°C. After the reaction mixture had been allowed to cool, the solvent was removed under reduced pressure. Ethyl acetate (90 mL) was added to the residue, and the mixture was heated to 40°C for dissolution. The organic solution (organic layer) was washed with water (30 mL × 2) and dried over sodium sulfate, and the solvent was removed under reduced pressure. Hexane (60 mL) was added to the residue, and the mixture was stirred for one hour at 25°C. The formed precipitates were collected through filtration and dried, to thereby yield 5.10 g of the title compound as yellow-orange crystals (95%).
¹H-NMR (270 MHz, CDCl₃) δ: 0.64-0.94 (4H, m), 1.27 (3H, t, J = 7.0 Hz), 1.45 (9H, s), 3.24-3.91 (5H, m), 3.85 (3H, s), 3.88(2H, s), 4.21 (2H, q, J = 7.0 Hz, 14.3 Hz), 6.27 (1H, dd, J = 7.0 Hz, 14.0 Hz), 7.57 (1H, dd, J = 7.0 Hz, 14.0 Hz)
MASS: m/e = 439 (EIMS)

### Example 6: Ethyl 3-[(1R,2S)-2-fluoro-1-cyclopropylamino]-2-[4-[7-(S)-tert-butoxycarbonylamino-5-azaspiro[2.4]hept-5-yl]-2,5-difluorobenzoyl]acrylate

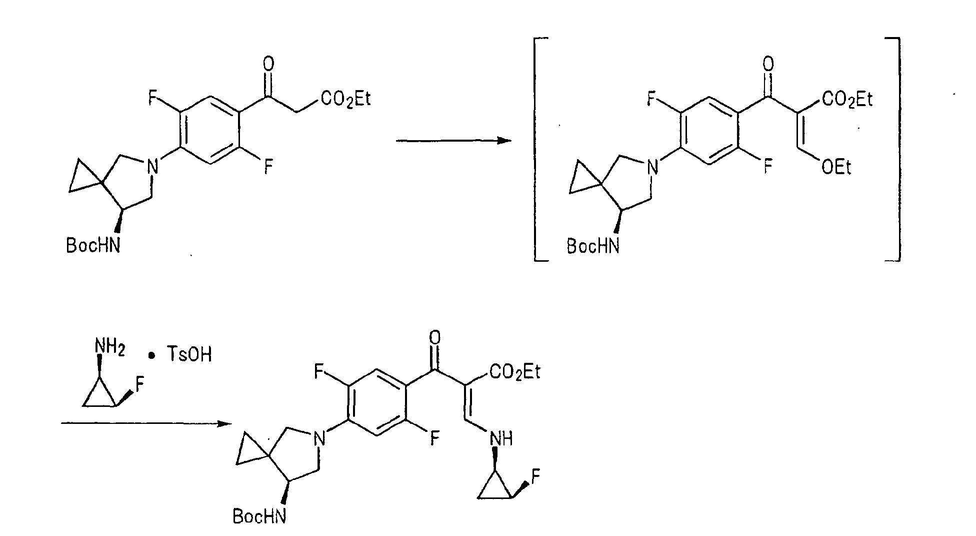

Ethyl 4-[7-(S)-tert-butoxycarbonylamino-5-azaspiro[2.4]hept-5-yl]-2,5-difluorobenzoylacetate (1.00 g, 2.29 mmol) was dissolved in acetic anhydride (2.16 mL, 10 eq.) and ethyl orthoformate (3.79 mL, 10 eq.), and the mixture was stirred for three hours at 130°C. After the reaction mixture was allowed to cool down, the solvent was removed under reduced pressure. Toluene (10 mL) was added to the residue, and the mixture was subjected to co-boiling twice. After completion of co-boiling, toluene (30 mL), triethylamine (0.38 mL, 1.2 eq.), and a (1R,2S)-2-fluorocyclopropylamine p-toluenesulfonic acid salt (564 mg, 1.0 eq.) was added to the co-boiling residue, and the mixture was stirred for 30 minutes at 25°C. The formed organic layer was washed with water (20 mL) and an aqueous sodium bicarbonate solution (10 mL, NaHCO₃, 1.0 g) and dried over sodium sulfate, and the solvent was removed under reduced pressure. The residue was subjected to silica gel column chromatography (eluent: hexane/ethyl acetate = 2/1), and several fractions were combined. The solvent of the combined fraction was removed under reduced pressure, to thereby yield 870 mg of the title compound as a pale yellow solid (73%).
¹H-NMR (270 MHz, CDCl₃) δ: 0.69-1.50 (6H, m), 1.14 (3H, t, J = 7.0 Hz), 1.39 (9H, s), 3.16-4.00 (6H, m), 4.12 (2H, q, J = 7.0 Hz, 14.0 Hz), 4.82 (1H, m), 6.14 (1H, dd, J = 7.0 Hz, 14.0 Hz), 7.15 (1H, dd, J = 7.0 Hz, 14.0 Hz), 8.10 (1H, d, J = 13.5 Hz)
MASS: m/e = 524 (FABMS)

### Example 7: 7-[7-(S)-tert-Butoxycarbonylamino-5-azaspiro[2.4]hept-5-yl]-6-fluoro-1-[2-(S)-fluoro-1-(R)-cyclopropyl]-1,4-dihydro-4-oxoquinoline-3-carboxylic acid

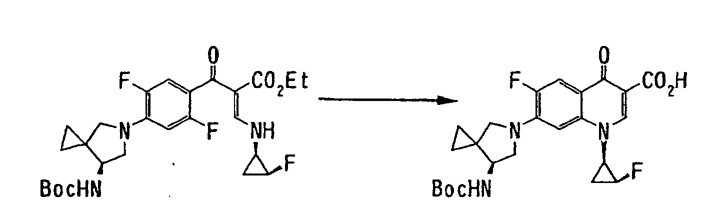

Tetrabutylammonium bromide (TBAB, 8 mg) was added to a mixture of ethyl 3-[(1R,2S)-2-fluoro-1-cyclopropylamino]-2-[4-[7-(S)-tert-butoxycarbonylamino-5-azaspiro[2.4]hept-5-yl]-2,5-difluorobenzoyl]acrylate (16) (524 mg, 1.00 mmol), toluene (10.0 mL), and 3N potassium hydroxide (2.0 mL, 6 eq.), and the resultant mixture was stirred for 15 hours at 50°C. After cooling of the mixture with ice, the mixture was slightly acidified with 3N aqueous hydrochloric acid, to thereby form a suspension. Water (15 mL) and saturated saline (5 mL) were added to the suspension so as to cause partition (poor partition performance). The aqueous layer was subjected to extraction with toluene (10 mL), to thereby recover organic components, and all the obtained organic layers were combined. The solvent was removed under reduced pressure. The residue was dissolved in ethyl acetate (50 mL) at 60°C, and water (20 mL) was added to the solution so as to cause partition. The organic layer was dried over sodium sulfate, and the solvent was removed under reduced pressure. Toluene (3 mL) and hexane (30 mL) were added to the residue, and the mixture was stirred for one hour at 25°C. The formed precipitates were collected through filtration and dried, to thereby yield 460 mg of the title compound as a brown solid (97%).
¹H-NMR (270 MHz, DMSO-d₆) δ: 0.64-1.78 (6H, m), 1.39 (9H, s), 3.13-3.98 (6H, m), 5.32 (1H, m), 6.95 (1H, d, J = 7.6 Hz), 7.82 (1H, d, J = 14.0 Hz), 8.66 (1H, s)
MASS: m/e = 476 (FARMS)

### Referential Example 2: 7-[7-(S)-Amino-5-azaspiro[2.4]hept-5-yl]-6-fluoro-1-[2-(S)-fluoro-1-(R)-cyclopropyl]-1,4-dihydro-4-oxoquinoline-3-carboxylic acid

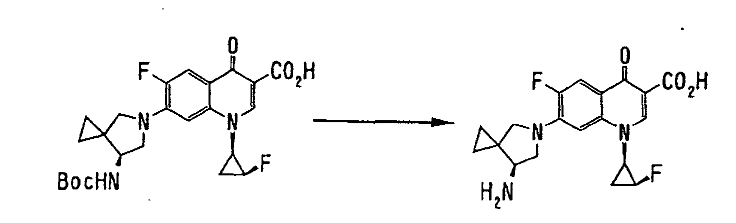

3N Hydrochloric acid (0.48 mL, 3 eq.) was added to a suspension of 7-[7-(S)-tert-butoxycarbonylamino-5-azaspiro[2.4]hept-5-yl]-6-fluoro-1-[2-(S)-fluoro-1-(R)-cyclopropyl]-1,4-dihydro-4-oxoquinoline-3-carboxylic acid (230 mg, 0.48 mmol) in isopropanol (1.1 mL), and the mixture was stirred for 1.5 hours at 70°C. After the mixture was cooled to 25°C, the mixture was neutralized through dropwise addition of triethylamine (0.20 mL, 3 eq.). The solvent was removed under reduced pressure, and acetonitrile (11.5 mL) and water (0.23 mL) were added to the residue, followed by stirring for one hour at 25°C. The formed precipitates were collected through filtration and dried, to thereby yield 102 mg of the title compound as pale brown crystals (56%).
¹H-NMR (270 MHz, D₂O-NaOD) : Identical with the ¹H-NMR spectrum of the title compound synthesized through a known method
MASS: Identical with the data of the title compound synthesized through a known method (m/e = 375; EIMS)

### Example 8: Ethyl 4-[7-(S)-acetylamimo-5-azaspiro[2.4]hept-5-yl]-2,5-difluoro-3-methoxybenzoylacetate

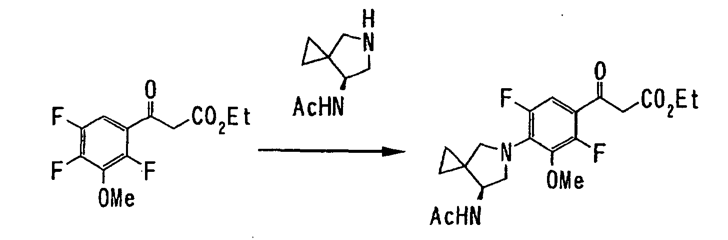

7-(S)-Acetylamimo-5-azaspiro[2.4]heptane (1.00 g, 1.05 eq.) was added to a solution containing ethyl 2,4,5-trifluoro-3-methoxybenzoylacetate (1.71 g, 6.17 mmol), acetonitrile (30 mL), and triethylamine (1.3 mL, 2.0 eq.), and the mixture was stirred for seven hours at 70°C. After the reaction mixture had been allowed to cool, the solvent was removed under reduced pressure. Ethyl acetate (68 mL) was added to the residue, and the resultant organic solution (organic layer) was washed with water (34 mL). The organic layer was dried over sodium sulfate, and hexane (30 mL) was added to the residue, followed by stirring for one hour at 25°C. The formed precipitates were collected through filtration and dried, to thereby yield 1.93 g of the title compound as pale yellow crystals (76%).
¹H-NMR (270 MHz, DMSO-d₆) δ: 0.56-0.86 (4H, m), 1.18 (3H, t, J = 7.2 Hz), 1.83 (3H, s), 3.45-4.04 (5H, m), 3.74 (3H, s), 3.94 (2H, d, J = 2.7 Hz), 4.11 (2H, q, J = 7.2 Hz, 14.4 Hz), 7.31 (1H, dd, J = 7.2 Hz, 15.4 Hz)
MASS: m/e = 411 (FABMS)

### Referential Example 3: Ethyl 2,4,5-trifluoro-3-methoxybenzoate

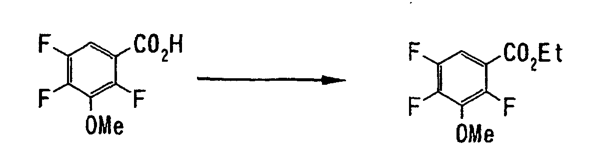

Sulfuric acid (3 mL) was added to a solution of 2,4,5-trifluoro-3-methoxybenzoic acid (30.00 g, 145.5 mmol) in ethanol (240 mL), and the mixture was stirred for 20 hours under reflux. After the reaction mixture was allowed to cool down, the solvent was removed under reduced pressure. Ethyl acetate (10 mL) was added to the residue, and the mixture was subjected to co-boiling. After completion of co-boiling, the residue was dissolved in ethyl acetate (150 mL), and the organic solution (organic layer) was washed with water (90 mL) (aqueous layer: acidic). The resultant organic layer was washed with sodium hydrogencarbonate (1 g)/water (100 mL) (aqueous layer: slightly basic), and the organic layer was further washed with saturated saline (50 mL). The thus-washed organic layer was dried over sodium sulfate, and the solvent was removed under reduced pressure, to thereby yield 31.01 g of the title compound as pale yellow oil (91%).
¹H-NMR (270 MHz, DMSO-d₆) δ: 1.33 (3H, t, J = 7.1 Hz), 4.02 (3H, s), 4.34 (2H, q, J = 7.1 Hz, 14.1 Hz), 7.58 (1H, m)

### Referential Example 4: Ethyl 4-[7-(S)-tert-butoxycarbonylamino-5-azaspiro[2.4]hept-5-yl]-2,5-difluoro-3-methoxybenzoate

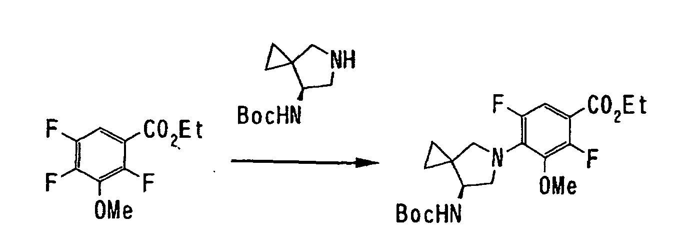

Potassium carbonate (4.70 g, 2.0 eq.) and 7-(S)-[tert-butoxycarbonylamino]-5-azaspiro[2.4]heptane (4.34 g, 1.2 eq.) were added to a solution of ethyl 2,4,5-trifluoro-3-methoxybenzoate (4.00 g, 17.00 mmol) in acetonitrile (40 mL), and the mixture was stirred for six hours under reflux. After the reaction mixture was allowed to cool down, the formed inorganic product was removed through filtration, and the solvent of the filtrate was removed under reduced pressure. Ethyl acetate (80 mL) was added to the residue, and the formed organic solution (organic layer) was washed with water (20 mL). The organic layer was dried over sodium sulfate, and the solvent was removed under reduced pressure, followed by co-boiling with hexane. Hexane (80 mL) was added to the residue, and the mixture was stirred for one hour at 25°C. The formed precipitates were collected through filtration and dried, to thereby yield 5.32 g of the title compound as colorless crystals (73%). The solvent of the crystallization mother liquor was removed under reduced pressure, and isopropyl alcohol (20 mL) and water (10 mL) were added to the residue, followed by stirring for one hour under ice-cooling. The formed precipitates were collected through filtration and dried, to thereby further yield 0.55 g of the title compound as colorless crystals (8%, total 81%). The solvent of the crystallization mother liquor was removed under reduced pressure. The residue was subjected to silica gel column chromatography (eluent: hexane/ethyl acetate = 5/1), and several fractions were combined. The solvent of the combined fraction was removed under reduced pressure, to thereby further yield 0.40 g of the title compound as colorless crystals (5%, total 86%).
¹H-NMR (270 MHz, DMSO-d₆) δ: 0.43-0.91 (4H,, m), 1.28 (3H, t, J = 7.2 Hz), 1.38 (9H, s), 3.38-4.06 (5H, m), 3.73 (3H, s), 4.25 (2H, q, J = 7.2 Hz, 14.3 Hz), 7.20 (1H, m), 7.25 (1H, q, J = 7.0 Hz, 15.0 Hz)
MASS: m/e = 427 (FABMS)

### Referential Example 5: 4-[7-(S)-tert-Butoxycarbonylamino-5-azaspiro[2.4]hept-5-yl]-2,5-difluoro-3-methoxybenzoic acid

3N Potassium hydroxide (4.0 mL, 1.08 eq.) was added to a solution of ethyl 4-[7-(S)-tert-butoxycarbonylamino-5-azaspiro[2.4]hept-5-yl]-2,5-difluoro-3-methoxybenzoate (4.75 g, 11.14 mmol) in ethanol (47.5 mL), and the mixture was stirred for two hours at 80°C. After cooling of the reaction mixture with ice, the mixture was neutralized through addition of 3N aqueous hydrochloric acid, and the solvent was removed under reduced pressure. Methanol (95 mL) was added to the residue, and the formed inorganic product was removed through filtration. The filtrate was dried over sodium sulfate, and the solvent was removed under reduced pressure, to thereby yield 5.19 g of the title compound as a white solid.
¹H-NMR (270 MHz, DMSO-d₆) δ: 0.47-0.91 (4H, m), 1.38 (9H, s), 3.31-3.85 (5H, m), 3.75 (3H, s), 7.25 (1H, q, J = 7.0 Hz, 14.6 Hz)
MASS: m/e = 398 (EIMS)

### Referential Example 6: Ethyl 2,5-difluoro-3-methoxy-4-[7-(S)-[1-(S)-phenylethyl]amino-5-azaspiro[2.4]hept-5-yl]benzoate

Potassium carbonate (9.40 g, 4.0 eq.) and a 7-(S)-[1-(S)-phenylethyl]amino-5-azaspiro[2.4]heptane oxalic acid salt (6.26 g, 1.2 eq.) were added to a solution of ethyl 2,4,5-trifluoro-3-methoxybenzoate (4.00 g, 17.00 mmol) in acetonitrile (60 mL), and the mixture was stirred for six hours undre reflux. After the reaction mixture was allowed to cool, the formed inorganic product was removed through filtration, and the solvent of the filtrate was removed under reduced pressure. Ethyl acetate (80 mL) was added to the residue, and the formed organic solution (organic layer) was washed with water (40 mL) and saturated saline (20 mL). The organic layer was dried over sodium sulfate, and the solvent was removed under reduced pressure, to thereby yield 7.99 g of the title compound as pale yellow oil.
¹H-NMR (270 MHz, CDCl₃) δ: 0.39-1.24 (4H, m), 1.24-1.55 (6H, m), 2.65 (1H, m), 3.37-4.02 (5H, m), 3.80 (3H, s), 4.39 (2H, q, J = 7.2 Hz, 14.4 Hz), 6.90-7.45 (6H, m)
MASS: m/e = 431 (FARMS)

### Referential Example 7: 2,5-Difluoro-3-methoxy-4-[7-(S)-[1-(S)-phenylethyl]amino-5-azaspiro[2.4]hept-5-yl]benzoic acid

3N Potassium hydroxide (5.3 mL, 1.1 eq.) was added to a solution of ethyl 2,5-difluoro-3-methoxy-4-[7-(S)-[1-(S)-phenylethyl]amino-5-azaspiro[2.4]hept-5-yl]benzoate (6.22 g, 14.45 mmol) in ethanol (93 mL), and the mixture was stirred for 1.5 hours at 80°C. After cooling of the reaction mixture with ice, the mixture was neutralized through addition of 1N hydrochloric acid (ethanolic), and the solvent was removed under reduced pressure. Ethyl acetate (50 mL) was added to the residue, and the formed organic solution (organic layer) was washed with water (50 mL × 2). The solvent was removed under reduced pressure, and hexane (50 mL) was added to the residue, followed by stirring for one hour at 25°C. The formed precipitates were collected through filtration and dried, to thereby yield 4.11 g of the title compound as colorless crystals (71%). The solvent of the crystallization mother liquor was removed under reduced pressure. The residue was dissolved in ethyl acetate (1 mL), and hexane (30 mL) was added thereto, followed by stirring for one hour at 25°C. The formed precipitates were collected through filtration and dried, to thereby further yield 0.83 g of the title compound as colorless crystals (14%, total 85%).
¹H-NMR (270 MHz, DMSO-d₆) δ: 0.45-0.91 (4H, m), 1.23-1.35 (6H, m), 2.59 (1H, m), 3.37-3.93 (5H, m), 3.80 (3H, s), 7.12-7.32 (6H, m); MASS: m/e = 403 (FABMS)

### Industrial Applicability

According to the present invention, the compounds represented by formula (2), which are useful as an antibacterial agent, can be produced having a high yield at low cost.

## Claims

1. A process for producing a compound represented by formula (2): 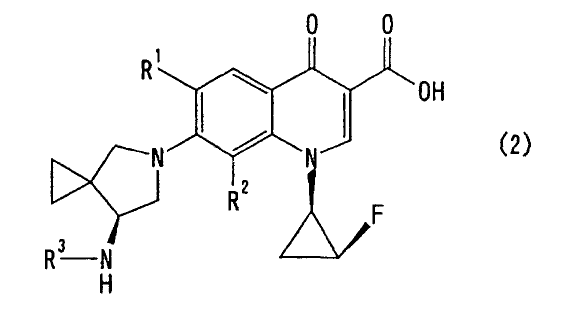 (wherein each of R¹ and R² independently represents a hydrogen atom, a fluorine atom, a chlorine atom, a C1-C6 alkyl group, or a C1-C6 alkoxy group; and R³ represents a hydrogen atom or an amino-group-protective group), the process comprising:
reacting a compound represented by formula (3): 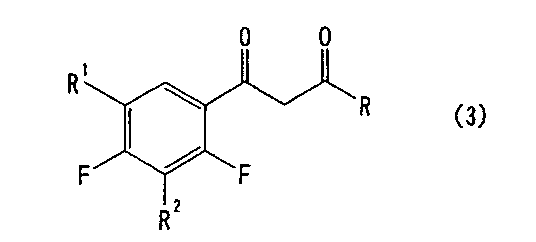 (wherein R represents an aryloxy group, an aralkyloxy group, or a C1-C6 alkoxy group; and R¹ and R² have the same meanings as defined above) with a compound represented by formula (4): 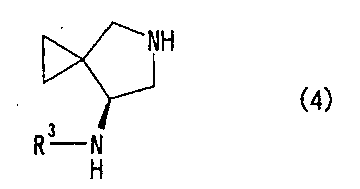 (wherein R³ has the same meaning as defined above), to thereby yield a compound represented by formula (5): 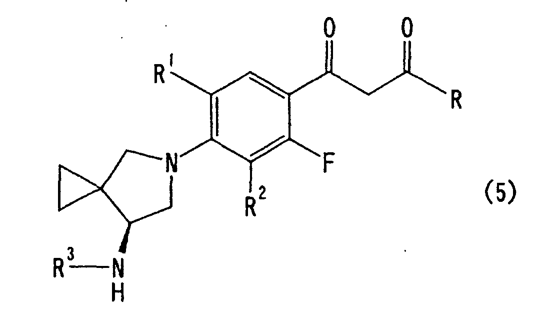 (wherein R, R¹, R², and R³ have the same meanings as defined above);
reacting the compound represented by formula (5) with an alkyl orthoformate, to thereby yield a compound represented by formula (6): 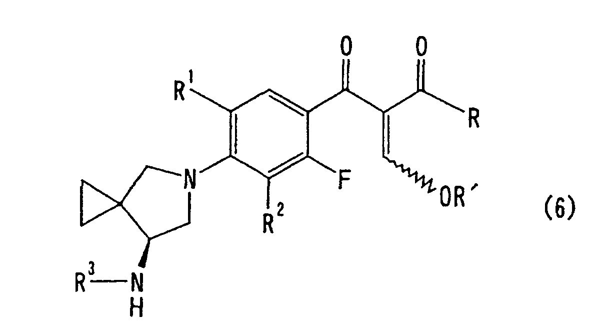 (wherein R' represents a C1-C6 alkyl group; and R, R¹ , R², and R³ have the same meanings as defined above);
reacting the compound represented by formula (6) with a compound represented by formula (7): 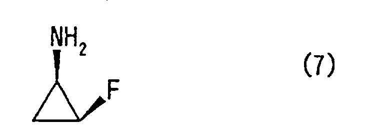 to thereby yield a compound represented by formula (8): 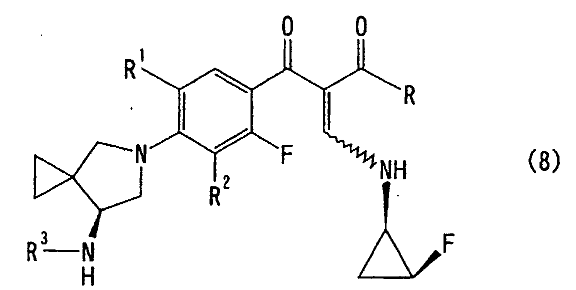 (wherein R, R¹, R², and R³ have the same meanings as defined above);
performing ring closure of the compound represented by formula (8); and
performing ester hydrolysis of the formed ring-closed compound.

2. A process as described in claim 1, wherein the compound represented by formula (4) is reacted in the presence of a base.

3. A process as described in claim 2, wherein the base is trimethylamine, triethylamine, 4-(dimethylamino)pyridine, ammonia, potassium carbonate, sodium carbonate, sodium hydroxide, or potassium hydroxide.

4. A process as described in claim 2, wherein the base is triethylamine.

5. A process as described in any one of claims 1 to 4, wherein the compound represented by formula (7) is reacted in the form of an acid-added salt thereof in the presence of a base.

6. A process as described in claim 5, wherein the salt of the compound represented by formula (7) is a salt thereof with hydrochloric acid, sulfuric acid, nitric acid, hydrofluoric acid, hydrobromic acid, hydriodic acid, p-toluenesulfonic acid, methanesulfonic acid, trifluoroacetic acid, acetic acid, formic acid, maleic acid, or fumaric acid.

7. A process as described in claim 5 or 6, wherein the base is trimethylamine, triethylamine, 4-(dimethylamino)pyridine, ammonia, potassium carbonate, sodium carbonate, sodium hydroxide, or potassium hydroxide.

8. A process as described in claim 5, wherein the salt of the compound represented by formula (7) is a p-toluenesulfonic acid salt and the base is triethylamine.

9. A process as described in any one of claims 1 to 8, wherein R¹ is a fluorine atom.

10. A process as described in any one of claims 1 to 9, wherein R² is a hydrogen atom.

11. A process as described in any one of claims 1 to 9, wherein R² is a methoxy group.

12. A process as described in any one of claims 1 to 11, wherein the amino-group-protective group represented by R³ is a group selected from the group consisting of an alkoxycarbonyl group, an aralkyloxycarbonyl group, an acyl group, an aralkyl group, an alkoxyalkyl group, and a substituted silyl group.

13. A process as described in claim 12, wherein the amino-group-protective group represented by R³ is a group selected from the group consisting of an aralkyloxycarbonyl group and an acyl group.

14. A process as described in claim 13, wherein R³ is a tert-butoxycarbonyl group or an acetyl group.

15. A process as described in any one of claims 1 to 14, wherein R is an ethoxy group or a methoxy group.

16. A process as described in any one of claims 1 to 15, wherein R' is an ethyl group or a methyl group.

17. A process for producing a compound represented by formula (2): 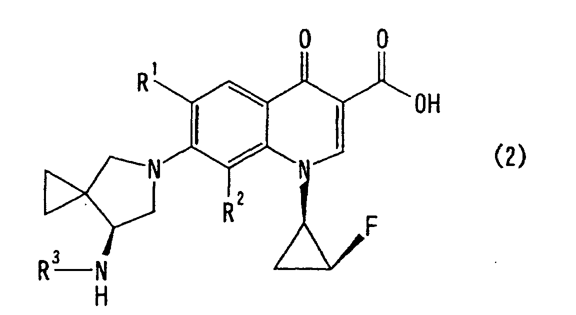 (wherein R¹, R², and R³ have the same meanings as defined above) comprising performing ring closure of a compound represented by formula (8): 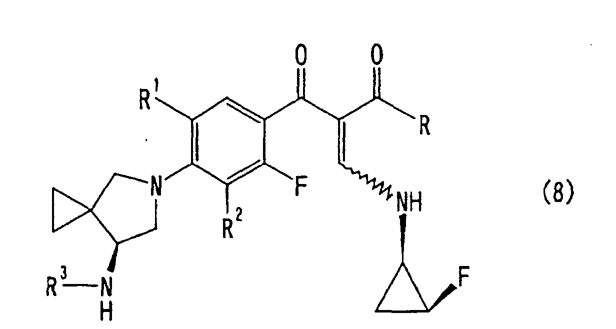 (wherein R, R¹, R², and R³ have the same meanings as defined above) and performing ester hydrolysis of the formed ring-closed compound.

18. A process as described in claim 17, wherein R¹ is a fluorine atom.

19. A process as described in claim 17 or 18, wherein R² is a hydrogen atom.

20. A process as described in claim 17 or 18, wherein R² is a methoxy group.

21. A process as described in any one of claims 17 to 20, wherein the amino-group-protective group represented by R³ is a group selected from the group consisting of an alkoxycarbonyl group, an aralkyloxycarbonyl group, an acyl group, an aralkyl group, an alkoxyalkyl group, and a substituted silyl group.

22. A process as described in claim 21, wherein the amino-group-protective group represented by R³ is a group selected from the group consisting of an aralkyloxycarbonyl group and an acyl group.

23. A process as described in claim 22, wherein R³ is a tert-butoxycarbonyl group or an acetyl group.

24. A process as described in any one of claims 17 to 23, wherein R is an ethoxy group or a methoxy group.

25. A compound represented by formula (5): 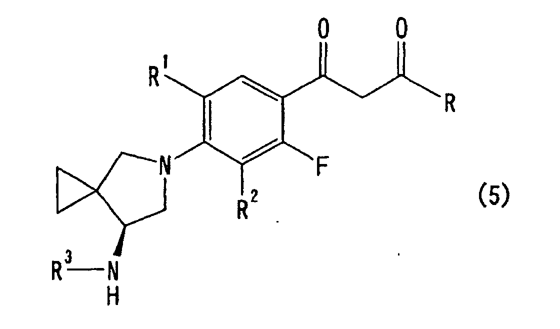 (wherein R represents an aryloxy group, an aralkyloxy group, or a C1-C6 alkoxy group; each of R¹ and R² independently represents a hydrogen atom, a fluorine atom, a chlorine atom, a C1-C6 alkyl group, or a C1-C6 alkoxy group; and R³ represents a hydrogen atom or an amino-group-protective group); a salt thereof; a hydrate of the compound (5); or a hydrate of the salt.

26. A compound, a salt thereof, a hydrate of the compound, or a hydrate of the salt as described in claim 25, wherein R¹ is a fluorine atom.

27. A compound, a salt thereof, a hydrate of the compound, or a hydrate of the salt as described in claim 25 or 26, wherein R² is a hydrogen atom.

28. A compound, a salt thereof, a hydrate of the compound, or a hydrate of the salt as described in claim 25 or 26, wherein R² is a methoxy group.

29. A compound, a salt thereof, a hydrate of the compound, or a hydrate of the salt as described in any one of claims 25 to 28, wherein R is an ethoxy group or a methoxy group.

30. A compound, a salt thereof, a hydrate of the compound, or a hydrate of the salt as described in any one of claims 25 to 29, wherein the amino-group-protective group represented by R³ is a group selected from the group consisting of an alkoxycarbonyl group, an aralkyloxycarbonyl group, an acyl group, an aralkyl group, an alkoxyalkyl group, and a substituted silyl group.

31. A compound, a salt thereof, a hydrate of the compound, or a hydrate of the salt as described in claim 30, wherein R³ is a group selected from the group consisting of an aralkyloxycarbonyl group and an acyl group.

32. A compound, a salt thereof, a hydrate of the compound, or a hydrate of the salt as described in any one of claims 25 to 28, wherein R³ is a tert-butoxycarbonyl group or an acetyl group.

33. A compound represented by formula (6): 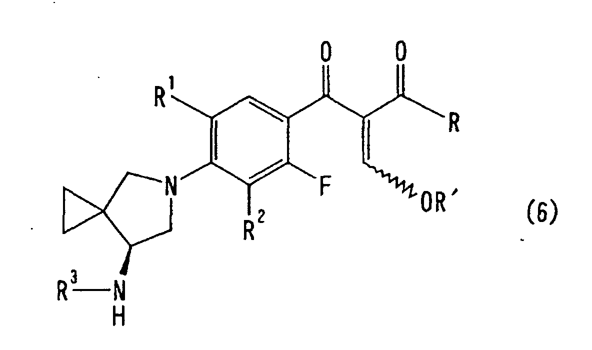 (wherein R represents an aryloxy group, an aralkyloxy group, or a C1-C6 alkoxy group; each of R¹ and R² independently represents a hydrogen atom, a fluorine atom, a chlorine atom, a C1-C6 alkyl group, or a C1-C6 alkoxy group; R³ represents a hydrogen atom or an amino-group-protective group; and R' represents a C2-C6 alkyl group); a salt thereof; a hydrate of the compound (6); or a hydrate of the salt.

34. A compound, a salt thereof, a hydrate of the compound, or a hydrate of the salt as described in claim 33, wherein R¹ is a fluorine atom.

35. A compound, a salt thereof, a hydrate of the compound, or a hydrate of the salt as described in claim 33 or 34, wherein R² is a hydrogen atom.

36. A compound, a salt thereof, a hydrate of the compound, or a hydrate of the salt as described in claim 33 or 34, wherein R² is a methoxy group.

37. A compound, a salt thereof, a hydrate of the compound, or a hydrate of the salt as described in any one of claims 33 to 36, wherein R is an ethoxy group or a methoxy group.

38. A compound, a salt thereof, a hydrate of the compound, or a hydrate of the salt as described in any one of claims 33 to 37, wherein R' is an ethyl group or a methyl group.

39. A compound, a salt thereof, a hydrate of the compound, or a hydrate of the salt as described in any one of claims 33 to 38, wherein the amino-group-protective group represented by R³ is a group selected from the group consisting of an alkoxycarbonyl group, an aralkyloxycarbonyl group, an acyl group, an aralkyl group, an alkoxyalkyl group, and a substituted silyl group.

40. A compound, a salt thereof, a hydrate of the compound, or a hydrate of the salt as described in claim 39, wherein R³ is a group selected from the group consisting of an aralkyloxycarbonyl group or an acyl group.

41. A compound, a salt thereof, a hydrate of the compound, or a hydrate of the salt as described in any one of claims 33 to 38, wherein R³ is a tert-butoxycarbonyl group or an acetyl group.

42. A compound represented by formula (8): 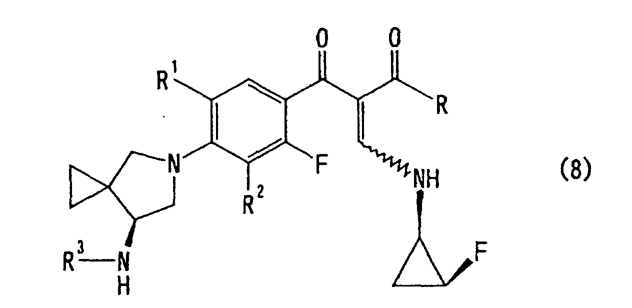 (wherein R represents an aryloxy group, an aralkyloxy group, or a C1-C6 alkoxy group; each of R¹ and R² independently represents a hydrogen atom, a fluorine atom, a chlorine atom, a C1-C6 alkyl group, or a C1-C6 alkoxy group; and R³ represents a hydrogen atom or an amino-group-protective group); a salt thereof; a hydrate of the compound (8); or a hydrate of the salt.

43. A compound, a salt thereof, a hydrate of the compound, or a hydrate of the salt as described in claim 42, wherein R¹ is a fluorine atom.

44. A compound, a salt thereof, a hydrate of the compound, or a hydrate of the salt as described in claim 42 or 43, wherein R² is a hydrogen atom.

45. A compound, a salt thereof, a hydrate of the compound, or a hydrate of the salt as described in claim 42 or 43, wherein R² is a methoxy group.

46. A compound, a salt thereof, a hydrate of the compound, or a hydrate of the salt as described in any one of claims 42 to 45, wherein R is an ethoxy group or a methoxy group.

47. A compound, a salt thereof, a hydrate of the compound, or a hydrate of the salt as described in any one of claims 42 to 46, wherein the amino-group-protective group represented by R³ is a group selected from the group consisting of an alkoxycarbonyl group, an aralkyloxycarbonyl group, an acyl group, an aralkyl group, an alkoxyalkyl group, and a substituted silyl group.

48. A compound, a salt thereof, a hydrate of the compound, or a hydrate of the salt as described in claim 47, wherein R³ is a group selected from the group consisting of an aralkyloxycarbonyl group and an acyl group.

49. A compound, a salt thereof, a hydrate of the compound, or a hydrate of the salt as described in any one of claims 42 to 46, wherein R³ is a tert-butoxycarbonyl group or an acetyl group.
